# EUROPEAN PATENT SPECIFICATION

(11) **EP 4 586 969 B1**
(45) Date of publication and mention of the grant of the patent: **01.04.2026**
(21) Application number: 23789815.0
(22) Date of filing: 15.09.2023
(51) Int. Cl.: A61F 2/95, A61F 2/24

(54) **CAPSULE LOADING ASSIST APPARATUSES AND ASSOCIATED METHODS**
KAPSELLADEUNTERSTÜTZUNGSVORRICHTUNGEN UND ZUGEHÖRIGE VERFAHREN
APPAREILS D'AIDE AU CHARGEMENT DE CAPSULE ET PROCÉDÉS ASSOCIÉS

(30) Priority: 16.09.2022 US 202263376031 P
(43) Date of publication of application: 23.07.2025
(73) Proprietor: Edwards Lifesciences Corporation, Irvine, CA 92614 (US)
(72) Inventor: SIROTE, Natanel Simcha, 3090163 Zikhron Ya'akov (IL)
(74) Representative: Eisenführ Speiser
(86) International application number: PCT/US2023/032853
(87) International publication number: WO 2024/059254

(56) References cited:
- EP-A1- 3 915 520
- US-A1- 2020 129 292
- US-A1- 2021 322 166

## Description

### CROSS REFERENCE TO RELATED APPLICATION

This application claims the benefit of U. S. Provisional Patent Application No. 63/376,031, filed September 16, 2022.

### FIELD

The present disclosure relates to implantable prosthetic heart valves and to apparatuses to assist in preparing delivery apparatuses that enclose such implantable prosthetic heart valves.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable.

In one specific example, a prosthetic heart valve can be mounted in a crimped state on the distal end of a delivery apparatus and advanced through the patient's vasculature (e.g., through a femoral artery and the aorta) until the prosthetic heart valve reaches the implantation site in the heart. The prosthetic heart valve is then expanded to its functional size, for example, by inflating a balloon on which the prosthetic valve is mounted, actuating a mechanical actuator that applies an expansion force to the prosthetic heart valve, or by deploying the prosthetic heart valve from a sheath of the delivery apparatus so that the prosthetic heart valve can self-expand to its functional size.

US 2021/0322166 A1 relates to devices, systems, and methods for intravascularly delivering an implantable device to a targeted anatomical site such as the mitral annulus. A delivery system includes a delivery member coupled to a handle assembly and extending distally from the handle assembly. A delivery catheter is concentrically positioned within an outer member and configured to advance the intravascular device relative to the outer member. The delivery catheter includes a distal can structure configured to house at least a portion of the intravascular device in a compressed, pre-deployed position.

### SUMMARY

Described herein are delivery apparatus and methods for implanting prosthetic heart valves. The disclosed apparatus and methods can, for example, facilitate loading a prosthetic heart valve in a delivery apparatus by exerting an axially distally directed force upon a delivery capsule of the delivery apparatus relative to a handle of the delivery apparatus as the delivery capsule is advanced in the distal direction to enclose the prosthetic heart valve. As such, the devices and methods disclosed herein can, among other things, overcome one or more of the deficiencies of typical prosthetic heart valves and their delivery apparatus.

A capsule loading assist device, according to the invention, comprises a lead screw and a loading assist nut engaged with the lead screw.

According to the invention, the loading assist nut comprises a capsule engagement surface configured to engage a proximal surface of a delivery capsule of a delivery apparatus to exert a distally directed loading assist force on the delivery capsule.

According to the invention, the capsule loading assist apparatus further comprises a torque transfer mechanism configured to convey a torque from a handle of the delivery apparatus to the lead screw to rotate the lead screw.

According to the invention, the capsule loading assist apparatus is configured such that rotating the lead screw in a first lead screw direction causes the loading assist nut to move along the lead screw in a distal direction.

A capsule loading assist assembly, according to the invention, comprises a delivery apparatus, a prosthetic heart valve supported by the delivery apparatus, and the capsule loading assist apparatus of the invention.

According to the invention, the delivery apparatus comprises a handle with a capsule translation knob, a delivery capsule, and a capsule shaft extending between the handle and the delivery capsule.

A method, according to the invention, comprises operatively coupling a delivery apparatus to a capsule loading assist apparatus. In addition to this step, a method further comprises the steps disclosed herein.

According to the invention, the delivery apparatus comprises a handle comprising a capsule translation knob, a delivery capsule, and a capsule shaft extending between the handle and the delivery capsule.

According to the invention, the capsule loading assist apparatus comprises a lead screw, a loading assist nut engaged with the lead and a torque transfer mechanism configured to convey a torque from the handle of the delivery apparatus to the lead screw to rotate the lead screw.

According to the invention, the method further comprises rotating the capsule translation knob in a first knob direction to translate the loading assist nut in a distal direction. The invention is set out in the appended set of claims.

The above devices can be used as part of an implantation procedure performed on a living animal or on a simulation, such as on a cadaver, cadaver heart, anthropomorphic ghost, simulator (e.g., with body parts, heart, tissue, etc. being simulated).

The various innovations of this disclosure can be used in combination or separately. This summary is provided to introduce a selection of concepts in a simplified form that are further described below in the detailed description. This summary is not intended to identify key features or essential features of the claimed subject matter, nor is it intended to be used to limit the scope of the claimed subject matter. The foregoing and other objects, features, and advantages of the disclosure will become more apparent from the following detailed description, claims, and accompanying figures.

### BRIEF DESCRIPTION OF THE DRAWINGS

FIG. 1 is a perspective view of a prosthetic heart valve according to an example, shown in a radially expanded state.
FIG. 2 is a side view of a delivery assembly comprising the prosthetic heart valve of FIG. 1 and a delivery apparatus, according to an example.
FIG. 3 is a schematic partially cross-sectional side view of a delivery assembly comprising a prosthetic heart valve and a delivery apparatus supported by a capsule loading assist apparatus, according to an example.
FIG. 4 is a partially cross-sectional side view of a delivery assembly supported by a capsule loading assist apparatus in an initial configuration, according to an example.
FIG. 5 is a partially cross-sectional side view of the delivery apparatus and the capsule loading assist apparatus of FIG. 4 in an intermediate configuration, according to an example.
FIG. 6 is a partially cross-sectional side view of the delivery apparatus and the capsule loading assist apparatus of FIG. 4 in a final configuration, according to an example.
FIG. 7 is a cross-sectional front view of the capsule loading assist apparatus of FIG. 4 as viewed along the line 7-7 in FIG. 4 and with a receiver arm of a loading assist nut in a closed position, according to an example.
FIG. 8 is a cross-sectional front view of the capsule loading assist apparatus of FIG. 7 with the receiver arm in an open position, according to an example.
FIG. 9 is a cross-sectional front view of the capsule loading assist apparatus of FIG. 4 as viewed along the line 9-9 in FIG. 4.

### DETAILED DESCRIPTION

### General Considerations

It should be understood that the disclosed examples can be adapted to deliver and implant prosthetic devices in any of the native annuluses of the heart (e.g., the pulmonary, mitral, and tricuspid annuluses), and can be used with any of various delivery approaches (e.g., retrograde, antegrade, transseptal, transventricular, transatrial, etc.).

For purposes of this description, certain aspects, advantages, and novel features of examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present or problems be solved.

Although the operations of some of the disclosed examples are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

As used herein, the term "approximately" and "about" means the listed value and any value that is within 10% of the listed value. For example, "about 1 mm" means any value between about 0.9 mm and about 1.1 mm, inclusive.

Directions and other relative references (e.g., inner, outer, upper, lower, etc.) may be used to facilitate discussion of the drawings and principles herein, but are not intended to be limiting. For example, certain terms may be used such as "inside," "outside," "top," "down," "interior," "exterior," and the like. Such terms are used, where applicable, to provide some clarity of description when dealing with relative relationships, particularly with respect to the illustrated examples. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or," as well as "and" and "or."

As used herein, "e.g." means "for example," and "i.e." means "that is."

As used herein, the term "simulation" means a performing an act on a cadaver, cadaver heart, anthropomorphic ghost, and/or a computer simulator (e.g., with the body parts, tissue, etc. being simulated).

### Introduction to the Disclosed Technology

Prosthetic valves disclosed herein can be radially compressible and expandable between a radially compressed state and a radially expanded state. Thus, the prosthetic valves can be crimped on or retained by an implant delivery apparatus in the radially compressed state during delivery, and then expanded to the radially expanded state once the prosthetic valve reaches the implantation site. It is to be understood that the prosthetic valves disclosed herein may be used with a variety of implant delivery apparatuses and can be implanted via various delivery procedures, examples of which will be discussed in more detail later.

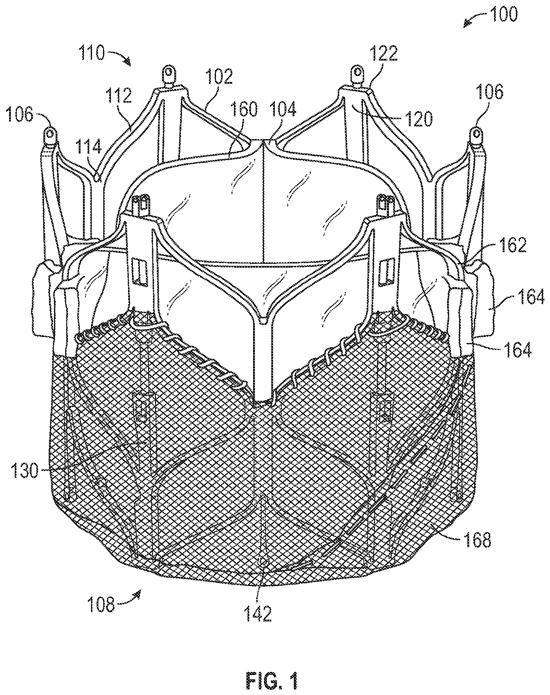

FIG. 1 shows an exemplary prosthetic heart valve 100, according to one example. Any of the prosthetic valves disclosed herein are adapted to be implanted in the native aortic annulus, although in other examples they can be adapted to be implanted in the other native annuluses of the heart (the pulmonary, mitral, and tricuspid valves). The disclosed prosthetic valves also can be implanted within vessels communicating with the heart, including a pulmonary artery (for replacing the function of a diseased pulmonary valve), the superior vena cava or the inferior vena cava (for replacing the function of a diseased tricuspid valve), or various other veins, arteries and vessels of a patient. The disclosed prosthetic valves also can be implanted within a previously implanted prosthetic valve (which can be a prosthetic surgical valve or a prosthetic transcatheter heart valve) in a valve-in-valve procedure.

In some examples, the disclosed prosthetic valves can be implanted within a docking or anchoring device that is implanted within a native heart valve or a vessel. For example, in one example, the disclosed prosthetic valves can be implanted within a docking device implanted within the pulmonary artery for replacing the function of a diseased pulmonary valve, such as disclosed in U.S. Publication No. 2017/0231756. In another example, the disclosed prosthetic valves can be implanted within a docking device implanted within or at the native mitral valve, such as disclosed in PCT Publication No. WO2020/247907. In another example, the disclosed prosthetic valves can be implanted within a docking device implanted within the superior or inferior vena cava for replacing the function of a diseased tricuspid valve, such as disclosed in U.S. Publication No. 2019/0000615.

### Delivery Techniques

For implanting a prosthetic valve within the native aortic valve via a transfemoral delivery approach, the prosthetic valve is mounted in a radially compressed state along the distal end portion of a delivery apparatus. The prosthetic valve and the distal end portion of the delivery apparatus are inserted into a femoral artery and are advanced into and through the descending aorta, around the aortic arch, and through the ascending aorta. The prosthetic valve is positioned within the native aortic valve and radially expanded (e.g., by inflating a balloon, actuating one or more actuators of the delivery apparatus, or deploying the prosthetic valve from a sheath to allow the prosthetic valve to self-expand). Alternatively, a prosthetic valve can be implanted within the native aortic valve in a transapical procedure, whereby the prosthetic valve (on the distal end portion of the delivery apparatus) is introduced into the left ventricle through a surgical opening in the chest and the apex of the heart and the prosthetic valve is positioned within the native aortic valve. Alternatively, in a transaortic procedure, a prosthetic valve (on the distal end portion of the delivery apparatus) is introduced into the aorta through a surgical incision in the ascending aorta, such as through a partial J-sternotomy or right parasternal minithoracotomy, and then advanced through the ascending aorta toward the native aortic valve.

For implanting a prosthetic valve within the native mitral valve via a transseptal delivery approach, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, into the right atrium, across the atrial septum (through a puncture made in the atrial septum), into the left atrium, and toward the native mitral valve. Alternatively, a prosthetic valve can be implanted within the native mitral valve in a transapical procedure, whereby the prosthetic valve (on the distal end portion of the delivery apparatus) is introduced into the left ventricle through a surgical opening in the chest and the apex of the heart and the prosthetic valve is positioned within the native mitral valve.

For implanting a prosthetic valve within the native tricuspid valve, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, and into the right atrium, and the prosthetic valve is positioned within the native tricuspid valve. A similar approach can be used for implanting the prosthetic valve within the native pulmonary valve or the pulmonary artery, except that the prosthetic valve is advanced through the native tricuspid valve into the right ventricle and toward the pulmonary valve/pulmonary artery.

Another delivery approach is a transatrial approach whereby a prosthetic valve (on the distal end portion of the delivery apparatus) is inserted through an incision in the chest and an incision made through an atrial wall (of the right or left atrium) for accessing any of the native heart valves. Atrial delivery can also be made intravascularly, such as from a pulmonary vein. Still another delivery approach is a transventricular approach whereby a prosthetic valve (on the distal end portion of the delivery apparatus) is inserted through an incision in the chest and an incision made through the wall of the right ventricle (typically at or near the base of the heart) for implanting the prosthetic valve within the native tricuspid valve, the native pulmonary valve, or the pulmonary artery.

In all delivery approaches, the delivery apparatus can be advanced over a guidewire previously inserted into a patient's vasculature. Moreover, the disclosed delivery approaches are not intended to be limited. Any of the prosthetic valves disclosed herein can be implanted using any of various delivery procedures and delivery devices known in the art.

Any of the systems, devices, apparatuses, etc. herein can be sterilized (for example, with heat/thermal, pressure, steam, radiation, and/or chemicals, etc.) to ensure they are safe for use with patients, and any of the methods herein can include sterilization of the associated system, device, apparatus, etc. as one of the steps of the method. Examples of heat/thermal sterilization include steam sterilization and autoclaving. Examples of radiation for use in sterilization include, without limitation, gamma radiation, ultra-violet radiation, and electron beam. Examples of chemicals for use in sterilization include, without limitation, ethylene oxide, hydrogen peroxide, peracetic acid, formaldehyde, and glutaraldehyde. Sterilization with hydrogen peroxide may be accomplished using hydrogen peroxide plasma, for example.

The treatment techniques, methods, steps, etc. described or suggested herein can be performed on a living animal or on a non-living simulation, such as on a cadaver, cadaver heart, anthropomorphic ghost, simulator (e.g., with the body parts, tissue, etc. being simulated), etc.

### Examples of the Disclosed Technology

FIG. 1 depicts one example of a prosthetic heart valve 100 which can be radially compressed for delivery through a patient's vasculature and radially expanded to a functional size at a desired implantation location within the patient' body (e.g., the native aortic valve). The prosthetic heart valve 100 comprises a frame 102 and a valvular structure 104.

The frame 102 (which can also be referred to as a stent or a support structure) can be configured to support the valvular structure 104 and for securing the prosthetic heart valve 100 within a native heart valve and/or within another support structure (e.g., an anchoring frame such as a coil) and/or a previously implanted prosthetic heart valve (i.e., in a valve-in-valve procedure). The frame 102 can further comprise one or more actuators 106 configured to radially expand or radially compress the frame 102, as further described in detail below.

With continued reference to FIG. 1, the frame 102 of the prosthetic heart valve 100 has a first end 108 and a second end 110. In the depicted orientation, the first end 108 of the frame 102 is an inlet end and the second end 110 of the frame 102 is an outlet end. In other examples, the first end 108 of the frame 102 can be the outlet end and the second end 110 of the frame 102 can be the inlet end. In some examples, the first end 108 also may be referred to as a distal end 108 of the prosthetic heart valve 100, and/or the second end 110 also may be referred to as a proximal end 110 of the prosthetic heart valve 100.

The frame 102 can comprise a plurality of interconnected angled struts 112 and vertical struts 114. In some examples, the angled struts 112 and the vertical struts 114 define a plurality of frame cells. As illustrated in FIG. 1, the vertical struts 114 can terminate axially inwards from both the first end 108 and the second end 110 of the frame 102.

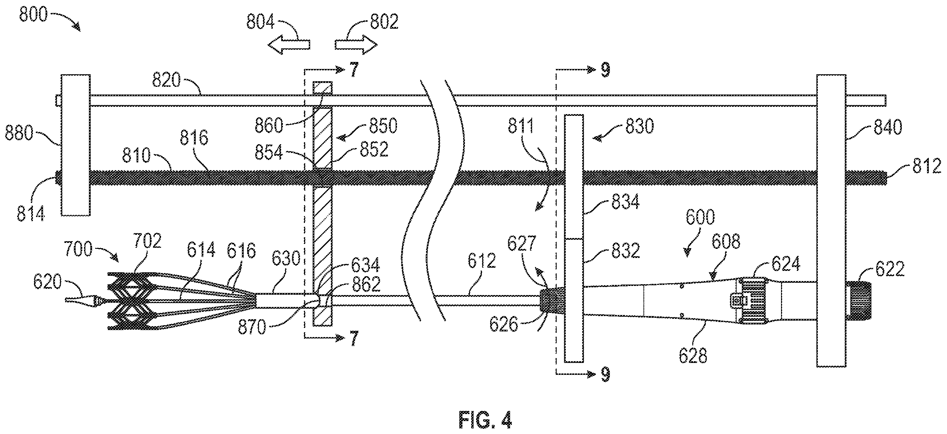

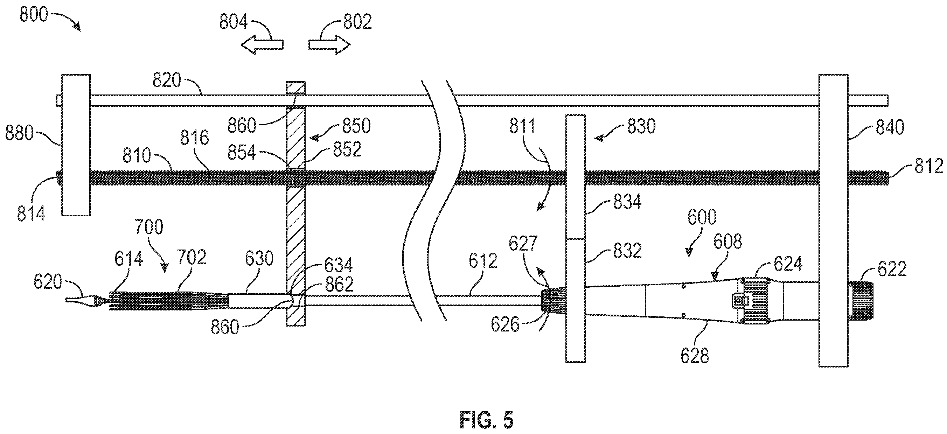

The frame 102 can be configured to move between a plurality of radial states, as shown, for example in FIGS. 4-5. For example, and as discussed in more detail below, FIG. 4 shows a frame 702 of a prosthetic heart valve 700 in a radially expanded state, while FIG. 5 shows the frame 702 in a partially radially compressed state. The depicted configurations are exemplary, and the frame 102 can be expanded or compressed to a lesser or greater extent than depicted. As the frame 102 moves between the various configurations, some of the struts 112, 114 of the frame 102 deflect or pivot relative to each other. For example, the angled struts 112 (which can also be referred to as "diagonal struts," i.e., the non-vertically and non-horizontally oriented struts) deflect relative to the vertically and horizontally oriented struts. In this manner, the frame 102 of the prosthetic heart valve 100 axially elongates when the frame is radially compressed and axially foreshortens when the frame 102 is radially expanded.

While the example prosthetic heart valves described above include mechanically expandable frames that are expanded by actuators 106, it is to be appreciated that in other examples, different frame expansion mechanisms could be used. For example, self-expanding, partially self-expanding, and balloon expandable frames 102 could be used in place of a mechanically actuated frame as previously described.

The frame 102 can be made of any of various suitable plastically-expandable materials (e.g., stainless steel, etc.) or self-expanding materials (e.g., Nitinol) as known in the art. When constructed of a plastically-expandable material, the frame 102 (and thus the valve 100) can be crimped to a radially compressed state on a delivery catheter and then expanded inside a patient by an inflatable balloon or equivalent expansion mechanism. When constructed of a selfexpandable material, the frame 102 (and thus the 100) can be crimped to a radially compressed state and restrained in the compressed state by insertion into a sheath or equivalent mechanism of a delivery catheter. Once inside the body, the valve can be advanced from the delivery sheath, which allows the valve to expand to its functional size.

Suitable plastically-expandable materials that can be used to form the frames disclosed herein (for example, the frame 102) include, metal alloys, polymers, or combinations thereof. Example metal alloys can comprise one or more of the following: nickel, cobalt, chromium, molybdenum, titanium, or other biocompatible metal. In some examples, the frame 102 can comprise stainless steel. In some examples, the frame 102 can comprise cobalt-chromium. In some examples, the frame 102 can comprise nickel-cobalt-chromium. In some examples, the frame 102 comprises a nickel-cobalt-chromium-molybdenum alloy, such as MP35N^{™} (tradename of SPS Technologies), which is equivalent to UNS R30035 (covered by ASTM F562-02). MP35N^{™}/UNS R30035 comprises 35% nickel, 35% cobalt, 20% chromium, and 10% molybdenum, by weight. Returning to FIG. 1, the valvular structure 104 of the prosthetic heart valve 100 can be coupled to the frame 102 (e.g., directly and/or indirectly via other components such a sealing skirt). The valvular structure 104 is configured to allow blood flow through the prosthetic heart valve 100 from the first end 108 (i.e., the inlet end) to the second end 110 (i.e., the outlet end) in an antegrade direction and to block blood from flowing through the prosthetic heart valve 100 from the second end 110 to the first end 108 in a retrograde direction. The valvular structure can include various components including a leaflet assembly comprising two or more leaflets 160. For example, the valvular structure 104 in the illustrated example comprises a leaflet assembly having three leaflets 160. It is to be understood, however, that in other examples, the valvular structure 104 could comprise a different number of leaflets.

The leaflets 160 of the prosthetic heart valve 100 can be made of a flexible material. For example, the leaflets 160 can be made from in whole or part, biological material, bio-compatible synthetic materials, or other such materials. Suitable biological material can include, for example, bovine pericardium, equine pericardium, porcine pericardium, and/or pericardium from other sources.

The leaflets 160 can be arranged to form commissures 162. The commissures 162 can, for example, be mounted to the frame at the commissure windows 140, as illustrated in FIG. 1. For example, each leaflet 160 can have two commissure tabs 164 on opposite sides of the leaflet 160. Each commissure tab 164 can be paired with an adjacent commissure tab 164 of an adjacent leaflet to form a respective commissure 162. Each pair of commissure tabs 164 can be coupled to a corresponding vertical strut 114 at a commissure window 140, such as by sutures or other fastening means. Each commissure 162 can include one or more reinforcing members, such as fabric reinforcing members, that are sutured to the commissure tabs 164 and/or the vertical struts 114 to reinforce the connection between the commissure tabs 164 and the vertical struts 114.

The inlet or cusp edge portions of the leaflets 160 can be coupled to the frame 102 via various techniques and/or mechanisms. For example, the cusp edge portions of the leaflets 160 can be sutured directly to selected angled struts 112 or vertical struts 114 located at the first end 108 (e.g., the inlet end) of the prosthetic heart valve. Alternatively, the cusp edge portions of the leaflets 160 can be sutured to an inner skirt (e.g., a fabric skirt, not shown), which in turn can be sutured to selected angled struts 112 or vertical struts 114 located at the first end 108 (e.g., the inlet end) of the prosthetic heart valve. The inlet portions of the leaflets 160 can also, in some examples, be coupled to the one or more axially extending suture posts 142 extending from selected vertical struts 114.

With continued reference to FIG. 1, the valvular structure 104 can further include an outer skirt or sealing member 166 disposed around the exterior of the frame 102. The outer skirt can be made of any suitable biocompatible and flexible material, including materials suitable for leaflets 160, or synthetic material, such as any suitable biocompatible fabric (e.g., polyethylene terephthalate (PET) fabric). The outer skirt 166 can be attached to the frame 102 by means of sutures, fabric, adhesive and/or other means for mounting, and in certain examples can be attached to the angled struts 112 and/or vertical struts 114 located at the first end 108 (e.g., the inlet end) of the prosthetic heart valve. The outer skirt 166 can be configured to improve the seal between the prosthetic heart valve 100 and the native heart valve in which the prosthetic heart valve has been implanted.

The skirt can be wholly or partly formed of any suitable biological material, synthetic material (for example, any of various polymers), or combinations thereof. In some examples, the skirt can comprise a fabric having interlaced yarns or fibers, such as in the form of a woven, braided, or knitted fabric. In some examples, the fabric can have a plush nap or pile. Exemplary fabrics having a plus nap or pile include velour, velvet, velveteen, corduroy, terrycloth, fleece, etc. In some examples, the skirt can comprise a fabric without interlaced yarns or fibers or randomly interlaced yarns or fibers, such as felt or an electrospun fabric. Exemplary materials that can be used for forming such fabrics (with or without interlaced yarns or fibers) include, without limitation, polyethylene (PET), ultra-high molecular weight polyethylene (UHMWPE), polytetrafluoroethylene (PTFE), expanded polytetrafluoroethylene (ePTFE), polyamide, etc. In some examples, the skirt can comprise a non-textile or non-fabric material, such as a film made from any of a variety of polymeric materials, such as PTFE, PET, polypropylene, polyamide, polyetheretherketone (PEEK), polyurethane (such as thermoplastic polyurethane (TPU)), etc. In some examples, the skirt can comprise a sponge material or foam, such as polyurethane foam. In some examples, the skirt can comprise natural tissue, such as pericardium (for example, bovine pericardium, porcine pericardium, equine pericardium, or pericardium from other sources).

Further details regarding prosthetic heart valves, including the valvular structure 104 and manner in which the valvular structure 104 can be coupled to the frame 102 of the prosthetic heart valve 100, can be found in U.S. Patent Nos. 6,730,118, 7,393,360, 7,510,575, 7,993,394, 8,652,202, and 9,393,110, U.S. Patent Application Publication No. 2018/0325665, and U.S. Application No. 63/138,890, filed January 19, 2021.

Described herein are examples of a steerable delivery apparatus (sometimes referred to as a steerable catheter) that can be used to navigate a subject's vasculature to deliver an implantable, expandable medical device (e.g., a prosthetic heart valve), tools, agents, or other therapy to a location within the body of a subject. Examples of procedures in which the steerable catheters are useful include neurological, urological, gynecological, fertility (e.g., in vitro fertilization, artificial insemination), laparoscopic, arthroscopic, transesophageal, transvaginal, transvesical, transrectal, and procedures including access in any body duct or cavity. Particular examples include placing implants, including stents, grafts, embolic coils, and the like; positioning imaging devices and/or components thereof, including ultrasound transducers; and positioning energy sources, for example, for performing lithotripsy, RF sources, ultrasound emitters, electromagnetic sources, laser sources, thermal sources, and the like.

A suitable delivery apparatus for implanting a prosthetic heart valve disclosed herein may comprise an elongated shaft configured to pass through the vasculature of a patient, one or more delivery actuators to manipulate a prosthetic heart valve within the patient's body, and a control mechanism by which a physician may control the actuators. Some examples of a delivery apparatus may further include a restraining mechanism configured to retain the prosthetic heart valve in a compressed configuration.

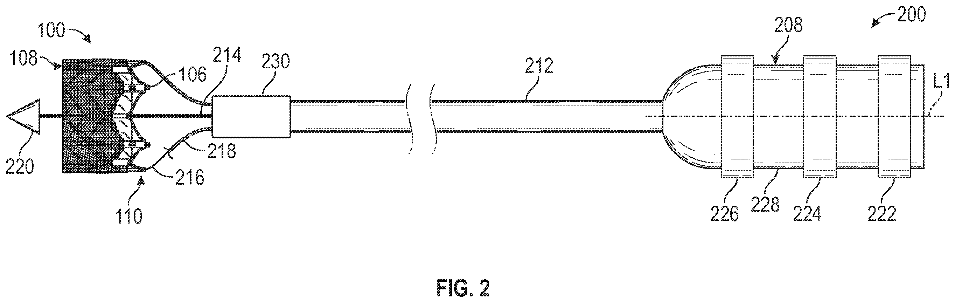

FIG. 2 illustrates a delivery apparatus 200, according to one example, designed to advance the prosthetic heart valve 100 through a patient's vasculature and/or to deliver the prosthetic heart valve 100 to an implantation site (e.g., native heart valve) within a patient's body. The prosthetic heart valve 100 can be mounted on, retained within, and/or releasably coupled to a distal end portion of the delivery apparatus 200.

The delivery apparatus 200 in the illustrated example generally includes a handle 208, a first shaft 212 (an outer shaft in the illustrated example) extending distally from the handle 208, a second shaft 214 (an inner shaft in the illustrated example) extending distally from the handle 208 through the first shaft 212, one or more delivery system actuators 216 extending distally through the outer shaft 212, and one or more support tubes (sometimes called support members) 218 that can extend distally through the outer shaft 212 and can abut the proximal end 110 of the prosthetic heart valve 100. The delivery apparatus 200 can further include a nose cone 220 connected to the distal end portion of the second shaft 214.

Each delivery system actuator 216 can have a distal end connected to an actuator 106 of the prosthetic heart valve 100. Each of the delivery system actuators 216 can extend through a respective support tube 218 and together can define a respective actuator assembly that can extend through the outer shaft 212 to the handle 208. In alternative examples, the delivery system actuators 216 and the support tubes 218 need not be co-axial with respect to each and instead can extend side-by-side through the shaft.

When the prosthetic heart valve 100 includes linear actuators 106, the delivery system actuators 216 and/or the support tubes 218 can be configured to radially expand the prosthetic heart valve 100 by bringing the ends 108, 110 of the prosthetic heart valve 100 closer together (i.e., squeezing the prosthetic heart valve 100 axially) thereby axially foreshortening and radially expanding the prosthetic heart valve 100. As one example, the delivery system actuators 216 can be configured to be actuated to provide a proximally directed (e.g., pulling) force to the actuators 106 of the prosthetic heart valve 100 while the one or more support tubes 218 can be configured to provide a countervailing distally directed (e.g., pushing) force to the proximal end 110 of the prosthetic heart valve 100. The actuators 106, in turn, may transmit the force to the distal end 108 of the prosthetic heart valve 100. In one such example, a physician can pull the delivery system actuators 216 to provide the proximally directed force to the distal end 108 of the prosthetic heart valve 100, while simultaneously gripping, holding, and/or pushing the handle 208 to provide the countervailing distally directed force to the proximal end 110 of the prosthetic heart valve 100.

When the prosthetic heart valve includes rotationally-driven actuators 106, the delivery system actuators 216 can be configured to apply a rotational force to the actuators 106. In such examples, the actuators 106 may have a first threaded end configured to connect with a corresponding threaded end of a delivery system actuator 216. When the delivery system actuator 216 is rotated in a first rotational direction, the actuator 106 can exert an axial force in the proximal direction on the distal end 108 of the prosthetic heart valve 100, thereby axially foreshortening and radially expanding the prosthetic heart valve 100. When the delivery system actuator 216 is rotated in a second rotational direction opposite to the first rotational direction, the actuator 106 can exert an axial force in the distal direction on the distal end 108 of the prosthetic heart valve 100, thereby axially extending and radially contracting the prosthetic heart valve 100. In such an example, a physician can rotationally manipulate the actuators 106 of the prosthetic heart valve 100 to radially expand or contract the prosthetic heart valve 100 to a desired diameter.

Although two pairs of delivery system actuators 216 and support tubes 218 are shown in FIG. 2, it should be understood that the delivery apparatus 200 can include more or less than three delivery system actuators 216 and/or three support tubes 218, in other examples. As just one example, the delivery apparatus 200 can include six delivery system actuators 216 and/or six support tubes 218. In other examples, a greater or fewer number of delivery system actuators 216 and/or support tubes 218 can be present, such as four, five, seven, and/or eight delivery system actuators 216 and/or four, five, seven, and/or eight support tubes 218. In some examples, the delivery apparatus 200 can include equal numbers of delivery system actuators 216 and support tubes 218. In other examples, however, the delivery apparatus 200 can include a different number of delivery system actuators 216 and support tubes 218.

As shown in FIG. 2, the handle 208 can include one or more knobs that can be configured to perform various functions of the delivery apparatus 200 to deliver the prosthetic heart valve 100 to an implantation location within a patient's body. In one example, the handle 208 can include a first knob 222, a second knob 224, and a third knob 226. In one example, the knobs 222, 224, 226 can be knobs that are rotatable about a longitudinal axis L1 of the handle 208 and relative to a body portion 228 of the handle.

In the example, the first knob 222 is located at a proximal end of the handle 208 and can be used to operate the delivery system actuators 216 of the delivery apparatus 200 and the actuators 106 of the prosthetic heart valve 100. The first knob 222 can be configured to operate a gearbox disposed within the handle 208. The delivery system actuators 216 can be coupled to the gearbox in order to be rotated by the gearbox. The rotation of the delivery system actuators 216 can be translated to rotational motion of the actuators 106 of the prosthetic heart valve 100. The second knob 224 can be configured to release the delivery system actuators 216 from the prosthetic heart valve 100 (e.g., after positioning the prosthetic heart valve 100 at the desired implantation location and expanding the prosthetic heart valve 100 to the working diameter).

In the example of FIG. 2, the third knob 226 is located at a distal end of the handle 208. The third knob 226 can be configured such that rotation of the knob relative to the handle body results in the first shaft 212 moving axially relative to the handle 208, the delivery system actuators 216, the prosthetic heart valve 100, and/or the second shaft 214.

As shown in FIG. 2, a delivery capsule 230 can be attached to a distal end of the first shaft 212. Axial movement of the first shaft 212 in a distal direction relative to the other shafts and prosthetic valve can move the delivery capsule 230 over the distal end portions of the delivery system actuators 216 and the prosthetic heart valve 100 (i.e., when the prosthetic heart valve 100 is in the radially compressed state) such that the prosthetic heart valve 100 is enclosed within the delivery capsule. Axial movement of the first shaft 212 in a proximal direction relative to the other shafts and the prosthetic valve can retract the delivery capsule 230 from the prosthetic heart valve 100, exposing the prosthetic heart valve, for example, for deployment at an implantation location. In some examples, the third knob 226 can be operatively coupled to a carriage within the distal portion of a cavity of the handle 208. The first shaft 212 can be coupled to the carriage such that movement of the carriage due to rotation of the third knob 226 results in axial displacement of the first shaft 212.

To prepare the delivery apparatus 200 and the prosthetic heart valve 100 for delivery to the desired implantation site, the delivery capsule 230 can be advanced in the distal direction relative to the prosthetic heart valve 100 to enclose the prosthetic heart valve 100 within the delivery capsule 230. In examples in which the delivery capsule 230 is coupled to and/or integral with the outer shaft 212, the delivery capsule 230 can be selectively advanced in the distal direction or retracted in the proximal direction by rotating the third knob 226 relative to the handle body 228. In particular, to advance delivery capsule 230 in the distal direction toward and/or over the prosthetic heart valve 100, the third knob 226 can be rotated in a first direction relative to the handle body 228.

In some examples, as the delivery capsule 230 advances in the distal direction, the delivery system actuators 216 and/or the prosthetic heart valve 100 may frictionally and/or mechanically resist the movement of the delivery capsule 230, necessitating that substantial force be applied to the delivery capsule 230 in the distal direction. In particular, advancing the delivery capsule 230 toward and/or over the prosthetic heart valve 100 also can operate to exert a radially inwardly directed force upon the delivery system actuators 216 and/or components of the prosthetic heart valve 100, such as the frame 102. Such forces thus may operate to radially contract the delivery system actuators 216 and/or the frame 102 as such components are mechanically constrained by the fixed inner diameter of the delivery capsule 230. The delivery system actuators 216 and/or components of the prosthetic heart valve 100 correspondingly may exert a proximally directed force on the delivery capsule 230 and/or on the first shaft 212 that must be overcome with a corresponding distally directed force in order to advance the delivery capsule 230 in the distal direction over the prosthetic heart valve 100.

In some examples, this distally directed force can be provided primarily or exclusively by the first shaft 212 to which the delivery capsule 230 is connected. In such cases, this force must be transferred along a full length of the outer shaft extending between the handle 208 and the delivery capsule 230. To facilitate this process, a user can manually maintain the outer shaft 212 in a substantially straight orientation and/or manually exert a tension force between the handle 208 and the delivery capsule 230.

In some examples, a capsule loading assist apparatus according to the present disclosure can be used to facilitate the process of advancing the delivery capsule 230 over the prosthetic heart valve 100. A capsule loading assist apparatus can, for example, facilitate maintaining the outer shaft 212 in a substantially straight orientation without manually gripping and/or supporting the handle 208 and/or the delivery capsule 230. As another example, a capsule loading assist apparatus can facilitate producing a tension force between the handle 208 and the delivery capsule 230 in a consistent and/or readily reproducible manner.

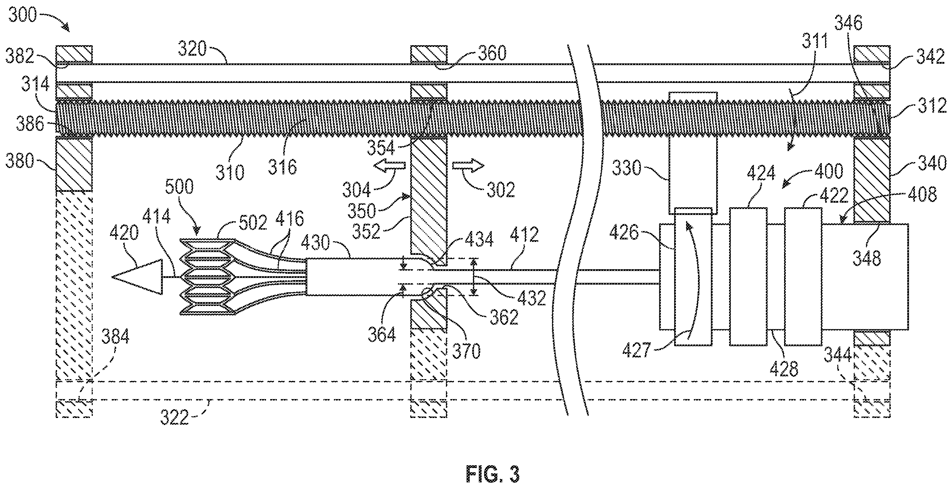

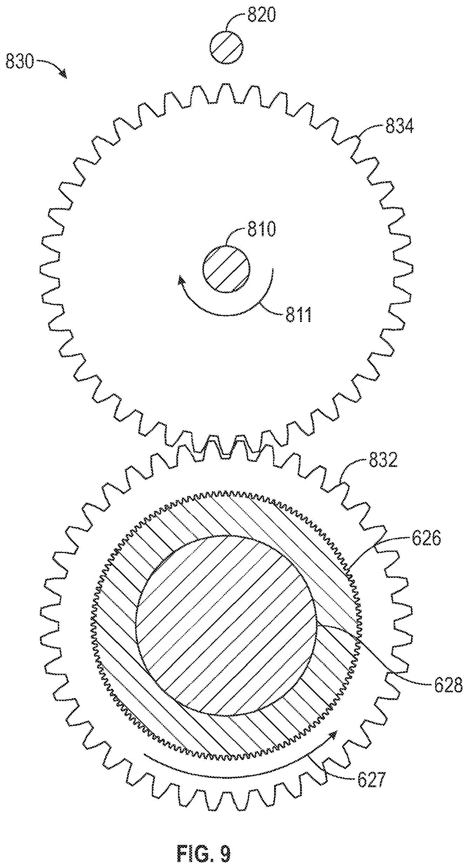

FIG. 3 illustrates an example of a capsule loading assist apparatus 300 that is coupled to (e.g., that supports) an example of a delivery apparatus 400. Additionally, and as described in more detail below, FIGS. 4-9 illustrate aspects of an example of a capsule loading assist apparatus 800 that can be coupled to an example of a delivery apparatus 600.

The delivery apparatus 400 and/or the delivery apparatus 600 can share any suitable components, features, attributes, etc. with the delivery apparatus 200 of FIG. 2. Additionally or alternatively, the delivery apparatus 200 of FIG. 2 and/or the delivery apparatus 600 of FIGS. 4-7 and 9 may be described as representing more specific examples of the delivery apparatus 400 schematically illustrated in FIG. 3. Accordingly, in the present disclosure, components with reference numerals beginning with a first digit of 2, 4, or 6 can share any suitable features, attributes, etc. with corresponding components beginning with another first digit of 2, 4, or 6 (i.e., components with reference numerals whose second and third digits are the same), even when such reference numerals are not specifically referenced herein. As an example, any two or more of the handle 208 of the delivery apparatus 200, the handle 408 of the delivery apparatus 400, and/or the handle 608 of the delivery apparatus 600 may be similar to and/or analogous to one another. As another example, any two or more of the delivery capsule 230, the delivery capsule 430, and/or the delivery capsule 630 may be similar to and/or analogous to one another.

Similarly, the capsule loading assist apparatus 800 of FIGS. 4-9 can share any suitable components, features, attributes, etc. with the capsule loading assist apparatus 300 of FIG. 3. Additionally or alternatively, the capsule loading assist apparatus 800 of FIGS. 4-9 may be described as representing a more specific example of the capsule loading assist apparatus 300 schematically illustrated in FIG. 3. Accordingly, in the present disclosure, components with reference numerals beginning with a first digit of 3 or 8 can share any suitable features, attributes, etc. with corresponding components beginning with the other first digit of 3 or 8 (i.e., components with reference numerals whose second and third digits are the same), even when such reference numerals are not specifically referenced herein. As an example, the lead screw 310 of the capsule loading assist apparatus 300 may be similar to and/or analogous to the lead screw 810 of the capsule loading assist apparatus 800.

In the example of FIG. 3, the delivery apparatus 400 is coupled to a prosthetic heart valve 500 that comprises a frame 502. For simplicity, FIG. 3 illustrates only the frame 502 of the prosthetic heart valve 500; however, it is to be understood that the prosthetic heart valve 500 can share any suitable components, features, attributes, etc. with any other prosthetic heart valve disclosed herein, such as the prosthetic heart valve 100 of FIGS. 1-2. FIG. 3 illustrates the prosthetic heart valve 500 in the radially expanded state.

Similar to the delivery apparatus 200 of FIG. 2, the delivery apparatus 400 of FIG. 3 comprises a handle 408 that can be selectively manipulated to selectively translate a delivery capsule 430 of the delivery apparatus relative to the prosthetic heart valve 500. In particular, in the example of FIG. 3, the handle 408 comprises a capsule translation knob 426 that can be rotated relative to a handle body 428 of the handle 408 to translate the delivery capsule 430 in a proximal direction 302 or in a distal direction 304. In this manner, the capsule translation knob 426 can be analogous to the third knob 226 of the delivery apparatus 200 of FIG. 2. As illustrated in FIG. 3, the handle 408 also can comprise a first knob 422 and a second knob 424, which can be analogous to the first knob 222 and the second knob 224, respectively, of the delivery apparatus 200 as described above.

As illustrated in FIG. 3, the capsule loading assist apparatus 300 comprises a lead screw 310 and a loading assist nut 350 engaged with the lead screw 310. The loading assist nut 350 comprises a capsule engagement surface 370 configured to engage a proximal surface 434 of the delivery capsule 430 of the delivery apparatus 400 to exert a distally directed force on the delivery capsule 430. In the present disclosure, the distally directed force exerted by the loading assist nut 350 upon the proximal surface 434 of the delivery capsule 430 also may be referred to as a loading force and/or as a loading assist force. As a result of the loading assist nut 350 exerting the loading assist force upon the delivery capsule 430, the loading assist nut 350 also may be described as exerting a tensioning force on the outer shaft 412 of the delivery apparatus 400 between the delivery capsule 430 and the handle 408.

The capsule loading assist apparatus 300 is configured such that rotating the lead screw 310 in a first lead screw direction 311 causes the loading assist nut 350 to move along the lead screw 310 in the distal direction 304 while the capsule engagement surface 370 remains in engagement with the delivery capsule 430. In particular, rotating the lead screw 310 while the handle 408 operates to advance the delivery capsule 430 in the distal direction 304 can provide a supplemental force (e.g., a distally directed force and/or a loading assist force) to facilitate moving the delivery capsule 430 over the prosthetic heart valve 500. For example, in order to move the delivery capsule 430 over the prosthetic heart valve 500, it may be helpful to exert a distally directed force between the delivery capsule 430 and the handle 408 that is sufficient to overcome a proximally directed force that is exerted upon the delivery capsule 430 by the components of the delivery apparatus 400 and/or of the prosthetic heart valve 500 that are enclosed by the delivery capsule 430. In this manner, the loading assist nut 350 can support a distal end of the outer shaft 412 to facilitate maintaining the outer shaft 412 in a substantially straight configuration, thereby preventing and/or reducing the likelihood of the outer shaft 412 buckling and/or kinking as the delivery capsule 430 is advanced over the prosthetic heart valve 500. Providing the loading assist force with the loading assist nut 350 of the capsule loading assist apparatus 300 as described herein thus can facilitate advancing the delivery capsule 430 over the prosthetic heart valve 500 in a stable and consistent manner.

As shown in FIG. 3, and as described in more detail below, the capsule loading assist apparatus 300 additionally comprises a torque transfer mechanism 330 that is configured to convey a torque from the handle 408 to the lead screw 310 to rotate the lead screw 310. Accordingly, actuating the handle 408 to advance the delivery capsule 430 in the distal direction 304 (e.g., by rotating the capsule translation knob 426 relative to the handle body 428) can, in some instances, additionally operate to rotate the lead screw 310 via the torque transfer mechanism 330 to move the loading assist nut 350 along the lead screw 310 in the distal direction 304. In this manner, the capsule loading assist apparatus 300 can be described as automatically advancing the loading assist nut 350 in the distal direction 304 as the handle 408 is actuated (e.g., via the knob 426) to move the delivery capsule 430 in the distal direction 304.

The loading assist nut 350 can have any of a variety of features and/or configurations for moving along the lead screw 410 and/or for engaging the delivery capsule 430 as described herein. In various examples, the loading assist nut 350 is configured to translate relative to the handle 408 without rotating relative to the handle 408 as the lead screw 410 rotates. In particular, the lead screw 310 can have a lead screw thread 316 that threadingly engages the loading assist nut 350 such that rotation of the lead screw 310 relative to the loading assist nut 350 causes the loading assist nut 350 to move along the lead screw 310.

As illustrated in FIG. 3, the loading assist nut 350 can comprise a main body 352 that defines a lead screw receiver 354 that receives the lead screw 310, as well as a capsule shaft receiver 362 configured to receive a capsule shaft of the delivery apparatus 400 that extends between the handle 408 and the delivery capsule 430. Specifically, in the example of FIG. 3, the capsule shaft is the outer shaft 412 of the delivery apparatus 400. As shown in FIG. 3, at least a portion of the main body 352 can extend between the lead screw receiver 354 and the capsule shaft receiver 362. The capsule shaft receiver 362 can be internally threaded so as to threadingly engage the lead screw thread 316 of the lead screw 310.

In some examples, the capsule loading assist apparatus 300 comprises one or more features to stabilize the loading assist nut 350 during operative use thereof For example, and as illustrated in FIG. 3, the capsule loading assist apparatus 300 can comprise a support shaft 320 that extends parallel to the lead screw 310 and that engages the loading assist nut 350 to restrict the loading assist nut 350 from rotating relative to the lead screw 310 and/or relative to the support shaft 320. In such examples, the loading assist nut 350 can comprise a support shaft receiver 360 that receives and engages the support shaft 320. Accordingly, when the lead screw 310 rotates, the engagement between the loading assist nut 350 and the support shaft 320 can restrict the loading assist nut 350 from rotating with the lead screw 310, thus forcing the loading assist nut 350 to instead translate axially (i.e., left or right in the depicted orientation) along the lead screw 310 due to the threaded connection therebetween. The added stability offered by the support shaft 320 also can serve to resist a rotation (e.g., a twisting) of the loading assist nut 350 out of a plane extending perpendicular to the lead screw 310.

In some examples, the support shaft receiver 360 is configured to slidingly engage the support shaft 320 as the loading assist nut 350 translates along the lead screw 310. For example, the support shaft receiver 360 and the support shaft 320 each can be unthreaded such that the support shaft receiver 360 operates as a plain bearing.

In some examples, the support shaft receiver 360 can engage the support shaft 320 in a threaded engagement. For example, each of the support shaft receiver 360 and the support shaft 320 can be threaded such that rotating the support shaft 320 concurrently with rotating the lead screw 310 causes the loading assist nut 350 to translate along each of the lead screw 310 and the support shaft 320.

In some examples, such as in the example of FIG. 3, the lead screw 310 extends between the support shaft 320 and the delivery apparatus 400 during operative use of the capsule loading assist apparatus 300. Stated differently, in such examples, during operative use of the capsule loading assist apparatus 300, the lead screw 310 and the support shaft 320 are positioned on opposite sides of the delivery apparatus 400. This is not required of all examples, however. For example, it additionally is within the scope of the present disclosure that the support shaft 320 can extend between the lead screw 310 and the delivery apparatus 400. For example, the capsule loading assist apparatus 300 can be configured such that the lead screw 310 and the delivery apparatus 400 are positioned on opposite sides of the support shaft 320 during operative use thereof. As another example, the capsule loading assist apparatus 300 can be configured such that the support shaft 320 and the lead screw 310 are positioned on opposite sides of the delivery apparatus 400 during operative use thereof.

In some examples, such as in the example of FIG. 3, the lead screw 310 and the support shaft 320 can be at least substantially coplanar with the delivery apparatus 400 (e.g., with the outer shaft 412) during operative use of the capsule loading assist apparatus 300. This is not required of all examples, however. For example, the capsule loading assist apparatus 300 can be configured such that the lead screw 310, the support shaft 320, and the outer shaft 412 of the delivery apparatus 400 are non-coplanar during operative use thereof. As a more specific example, the capsule loading assist apparatus 300 can be configured such that the lead screw 310, the support shaft 320, and the outer shaft 412 of the delivery apparatus 400 extend along respective axes that are substantially parallel and arranged in a triangular configuration during operative use thereof.

In some examples, the capsule loading assist apparatus 300 can comprise multiple support shafts. For example, and as illustrated in dashed lines in FIG. 3, the capsule loading assist apparatus 300 further can comprise a second support shaft 322 that engages the loading assist nut 350. In such examples, the second support shaft 322 can engage the loading assist nut 350 in any manner disclosed herein with respect to the engagement between the support shaft 320 (which also can be referred to as a first support shaft 320) and the loading assist nut 350. In some such examples, and as illustrated in FIG. 3, the delivery apparatus 400 extends between the first support shaft 320 and the second support shaft 322 during operative use of the capsule loading assist apparatus 300. In particular, supporting the loading assist nut 350 with support shafts positioned on either side of the delivery apparatus 400 can enhance a torsional stability of the loading assist nut 350 by mitigating the torque exerted by the loading assist nut 350 upon the lead screw 310 and/or the first support shaft 320 as the loading assist nut 350 is urged into engagement with the delivery capsule 230.

In some examples, such as in the example of FIG. 3, the main body 352 can define at least a portion of the capsule engagement surface 370 and/or of the capsule shaft receiver 362. In the present disclosure, the capsule shaft receiver 362 generally refers to an opening (e.g., a hole, an aperture, a notch, etc.) through which the capsule shaft extends during operative use of the capsule loading assist apparatus 300. Additionally, in the present disclosure, the capsule engagement surface 370 generally refers to a surface of the loading assist nut 350 that directly engages the delivery capsule 430 during operative use of the capsule loading assist apparatus 300. In some examples, the capsule engagement surface 370 at least substantially surrounds the capsule shaft receiver 362. Additionally or alternatively, the capsule engagement surface 370 can at least partially define the capsule shaft receiver 362.

The capsule shaft receiver 362 and/or the capsule engagement surface 370 can be configured to receive and/or interface with the delivery capsule 430 and/or the capsule shaft in any suitable manner. In some examples, and as illustrated in FIG. 3, the delivery capsule 430 has a delivery capsule diameter 432, and the capsule shaft receiver 362 has a capsule shaft receiver diameter 364 that is smaller than the delivery capsule diameter 432. Accordingly, when the capsule shaft receiver 362 receives the capsule shaft, translating the loading assist nut 350 in the distal direction 304 can urge the capsule engagement surface 370 against the proximal surface 434 of the delivery capsule 430.

The capsule engagement surface 370 also can have any suitable configuration for engaging the proximal surface 434 of the delivery capsule 430. As examples, the capsule engagement surface 370 can comprise a ramped surface, a concave surface, and/or a surface that is shaped to match a shape of the delivery capsule 430 (e.g., a convex shape of the proximal surface 434). Accordingly, in some such examples, the capsule engagement surface 370 can be configured to contact the delivery capsule 430 and/or the proximal surface 434 across a twodimensional contact area (e.g., as opposed to at a single point of contact and/or a onedimensional line of contact).

The capsule loading assist apparatus 300 further can comprise additional stabilizing structures that are spaced apart from the loading assist nut 350. For example, in some examples, and as illustrated in FIG. 3, the capsule loading assist apparatus 300 can comprise a proximal bearing 340 that supports a proximal end portion 312 of the lead screw 310 and/or a distal bearing 380 that supports a distal end portion 314 of the lead screw 310. In this manner, the proximal bearing 340 and/or the distal bearing 380 can support the lead screw 310 and/or the loading assist nut 350 (e.g., via the lead screw 310) relative to the delivery apparatus 400.

The proximal bearing 340 can be configured to engage the lead screw 310 such that the lead screw 310 can rotate relative to the proximal bearing 340. For example, and as illustrated in FIG. 3, the proximal bearing 340 can comprise a proximal bearing lead screw receiver 346 that receives the lead screw 310 and/or that engages the lead screw 310 as the lead screw 310 rotates relative to the proximal bearing 340. The proximal bearing 340 can engage the lead screw 310 via any suitable bearing surface. As examples, the proximal bearing lead screw receiver 346 can comprise a plain bearing, a ball bearing, a roller bearing, etc.

Similarly, the distal bearing 380 can be configured to engage the lead screw 310 such that the lead screw 310 can rotate relative to the distal bearing 380. For example, and as illustrated in FIG. 3, the distal bearing 380 can comprise a distal bearing lead screw receiver 386 that receives the lead screw 310 and/or that engages the lead screw 310 as the lead screw 310 rotates relative to the distal bearing 380. The distal bearing 380 can engage the lead screw 310 via any suitable bearing surface. As examples, the distal bearing lead screw receiver 386 can comprise a plain bearing, a ball bearing, a roller bearing, etc.

In some examples, and as illustrated in FIG. 3, the proximal bearing 340 and/or the distal bearing 380 additionally engages the (first) support shaft 320 and/or the second support shaft 322. In particular, in an example in which the capsule loading assist apparatus 300 comprises the (first) support shaft 320, the proximal bearing 340 can comprise a proximal bearing (first) support shaft receiver 342 that receives a proximal end portion of the (first) support shaft 320. Additionally or alternatively, in such an example, the distal bearing 380 can comprise a distal bearing (first) support shaft receiver 382 that receives a distal end portion of the (first) support shaft 320. In some examples, the proximal bearing (first) support shaft receiver 342 and/or the distal bearing (first) support shaft receiver 382 is fixedly coupled to the (first) support shaft 320.

Additionally or alternatively, in an example in which the capsule loading assist apparatus 300 comprises the second support shaft 322, the proximal bearing 340 can comprise a proximal bearing second support shaft receiver 344 that receives a proximal end portion of the second support shaft 322. Additionally or alternatively, in such an example, the distal bearing 380 can comprise a distal bearing second support shaft receiver 384 that receives a distal end portion of the second support shaft 322. In some examples, the proximal bearing second support shaft receiver 344 and/or the distal bearing second support shaft receiver 384 is fixedly coupled to the second support shaft 322.

In some examples, the proximal bearing 340 additionally is configured to engage and/or support the handle 408 of the delivery apparatus 400 relative to the lead screw 310. For example, and as illustrated in FIG. 3, the proximal bearing 340 can comprise a proximal bearing handle receiver 348 that is configured to receive the handle 408 to support the handle 408 relative to the lead screw 310. In some examples, the proximal bearing handle receiver 348 is configured to fixedly engage the handle 408, such as to restrict the handle 408 from rotating and/or translating relative to the proximal bearing 340.

In some examples, the lead screw 310 and/or the support shaft 320 can comprise one or more retention features (either integrally formed or coupled thereto) to retain the components between the proximal bearing 340 and the distal bearing 380. For example, the lead screw 310 and/or the support shaft 320 can each comprise one or more flanges that are larger than the openings in the bearings. The flanges can thereby limit axial movement of the lead screw 310 and/or the support shaft 320 relative to the bearings 340, 380. In some instances, the flanges can be a washer that this coupled to the lead screw 310 and/or the support shaft 320 with a fastener (and/or other coupling means).

The torque transfer mechanism 330 can have any suitable components and/or functionality for rotating the lead screw 310 responsive to rotation of a portion of the handle 408. As discussed above, and as illustrated in FIG. 3, the handle 408 can comprise a handle body 428 and a capsule translation knob (e.g., the third knob 426). That is, as used herein, the third knob 426 also may be referred to as a capsule translation knob 426. The capsule translation knob 426 is configured to be selectively rotated relative to the handle body 428 in a first knob direction 427 to translate the delivery capsule 430 in the distal direction 304 relative to the handle body 428. In some examples, the torque transfer mechanism 330 is configured to be selectively coupled to the capsule translation knob 426 such that rotation of the capsule translation knob 426 causes the lead screw 310 to rotate.

In some examples, the torque transfer mechanism 330 is configured to rotate the lead screw 310 such that, when the capsule translation knob 426 is rotated, the lead screw 310 translates the loading assist nut 350 at a rate that is equal to, or approximately equal to, a rate at which the capsule translation knob 426 advances the delivery capsule 430. In particular, the delivery apparatus 400 can be configured such that rotating the capsule translation knob 426 in the first knob direction 427 causes the delivery capsule 430 to translate in the distal direction 304 at a capsule advancement rate. Similarly, the torque transfer mechanism 330 can be configured such that rotation of the capsule translation knob 426 in the first knob direction 427 causes the lead screw 310 to rotate at a rotational rate such that the loading assist nut 350 translates in the distal direction 304 at a nut advancement rate that is equal to, or approximately equal to, the capsule advancement rate. The capsule advancement rate and the nut advancement rate each can be measured and/or quantified in any suitable manner, such as in terms of a translation distance along the distal direction 304 that is achieved for each full rotation of the capsule translation knob 426.

The torque transfer mechanism 330 can transfer torque from the capsule translation knob 426 to the lead screw 310 in any of a variety of manners. As discussed above, the capsule loading assist apparatus 300 is configured such that rotating the lead screw 310 in the first lead screw direction 311 causes the loading assist nut 350 to move along the lead screw 310 in the distal direction 304. In some examples, the first knob direction can be the same as the first lead screw direction. In other examples, such as in the example of FIG. 3, the first knob direction 627 can be opposite to the first lead screw direction 311.

In some examples, the torque transfer mechanism 330 is configured such that rotation of the capsule translation knob 326 at a knob rotational rate causes the lead screw 310 to rotate at a lead screw rotational rate that is equal to, or approximately equal to, the knob rotational rate. In such examples, the torque transfer mechanism 330 can be described as operatively interconnecting the capsule translation knob 326 and the lead screw 310 with a gear ratio (or an effective gear ratio) of 1:1. For example, the capsule translation knob 326 can be operatively coupled to a capsule shaft (e.g., the outer shaft 412) of the delivery apparatus 400 that extends between the handle 408 and the delivery capsule 430 via a screw mechanism with a capsule shaft screw pitch, and the lead screw thread 316 can have a lead screw pitch that is equal to, or approximately equal to, the capsule shaft screw pitch. This is not required of all examples, however, and it additionally is within the scope of the present disclosure that rotation of the capsule translation knob 326 at the knob rotational rate causes the lead screw to rotate at a lead screw rotational rate that is less than or greater than the knob rotational rate.

The torque transfer mechanism 330 can comprise any suitable mechanism for rotating the lead screw 310 responsive to rotation of the capsule translation knob 426, examples of which include one or more gears, belts, chains, and/or any other suitable mechanisms for rotationally coupling the capsule translation knob 426 and the lead screw 310.

As discussed above, FIGS. 4-9 illustrate aspects of the capsule loading assist apparatus 800, which can be coupled to the delivery apparatus 600. In particular, FIGS. 4-6 illustrate a sequence of configurations of the delivery apparatus 600 and the capsule loading assist apparatus 800 as the lead screw 810 is advanced in the distal direction 804 in engagement with the delivery capsule 630. In the example of FIGS. 4-6, the delivery apparatus 600 is coupled to a prosthetic heart valve 700 that comprises a frame 702 (visible in FIGS. 4-5). For simplicity, FIGS. 4-5 illustrate only the frame 702 of the prosthetic heart valve 700; however, it is to be understood that the prosthetic heart valve 700 can share any suitable components, features, attributes, etc. with any other prosthetic heart valve disclosed herein, such as the prosthetic heart valve 100 of FIGS. 1-2 and/or the prosthetic heart valve 500 of FIG. 3.

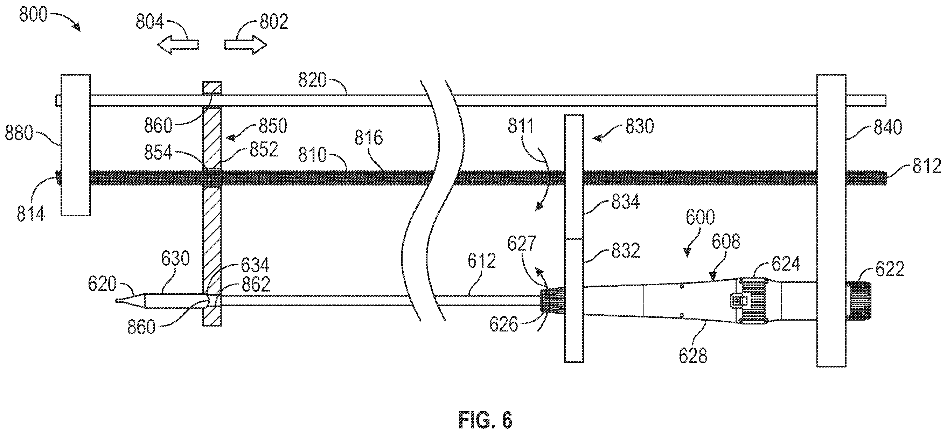

As shown in FIGS. 4-6, rotating the capsule translation knob 626 in the first knob direction 627 relative to the handle body 628 operates to move the loading assist nut 850 along the lead screw 810 in the distal direction 804 to exert a distally directed loading assist force on the delivery capsule 630 as the delivery capsule 630 also moves in the distal direction 804. As the delivery capsule 630 moves in the distal direction 804, the prosthetic heart valve 700 radially contracts from the radially expanded state (FIG. 4) to an intermediate state (FIG. 5) and to the radially compressed state (FIG. 6) in which the delivery capsule 630 can fully envelop the prosthetic heart valve 700.

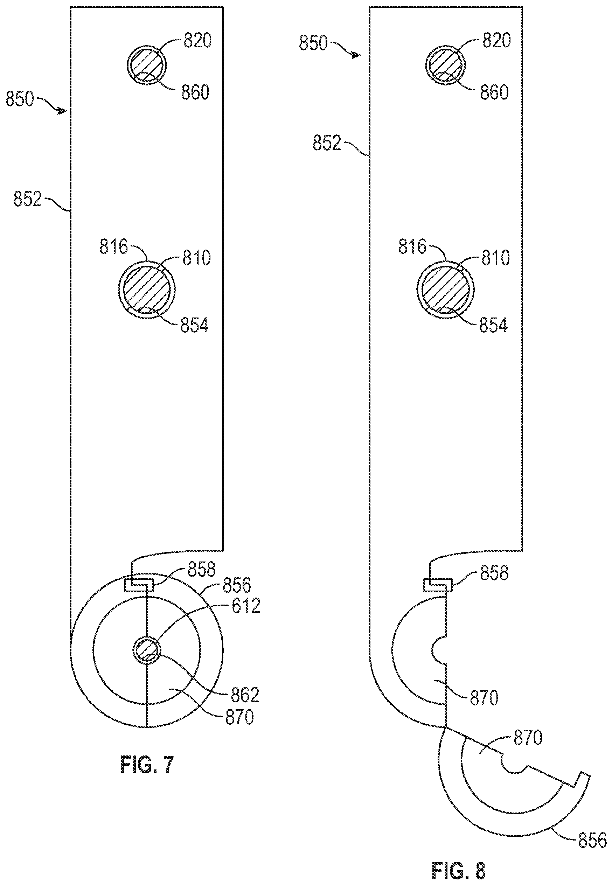

FIGS. 7-8 illustrate aspects of the loading assist nut 850 of the capsule loading assist apparatus 800. With reference to FIG. 3, the dimensions of the loading assist nut 350 (e.g., the capsule shaft receiver diameter 364 of the capsule shaft receiver 362) can be configured such that the loading assist nut cannot be longitudinally slid onto or off of the outer shaft 412 of the delivery apparatus 400 while the delivery capsule 430 and the handle 408 are attached to the outer shaft 412. Accordingly, the loading assist nut 350 can be configured to be selectively transitioned between distinct configurations to permit the outer shaft 412 to be inserted into or removed from the capsule shaft receiver 362. For example, with reference to the loading assist nut 850 of FIGS. 7-8, the loading assist nut 850 can comprise a moveable receiver arm 856 that is coupled to the main body 852 and that defines at least a portion of the capsule shaft receiver 862.

As shown in FIGS. 7-8, the receiver arm 856 is configured to be selectively transitioned between an open position (FIG. 8) and a closed position (FIG. 7). When the receiver arm 856 is in the open position, at least a portion of the receiver arm 856 is spaced away from (e.g., flexed and/or pivoted away from) the main body 852 to allow the capsule shaft to be inserted into or removed from the loading assist nut 850. When the receiver arm 856 is in the closed position, the loading assist nut 850 is operative to exert the loading assist force on the delivery capsule when the capsule shaft is received within the capsule shaft receiver 862.

In some examples, the capsule shaft receiver 862 can be configured and/or oriented to substantially surround the capsule shaft only when the receiver arm 856 is in the closed position. In some examples, and as illustrated in FIGS. 7-8, the receiver arm 856 defines at least a portion of the capsule engagement surface 870.

The receiver arm 856 can be configured to transition between the open position and the closed position in any suitable manner. In some examples, such as in the example of FIGS. 7-8, the receiver arm 856 is hingedly coupled to the main body 852. Additionally or alternatively, the receiver arm 856 can be configured to resiliently flex relative to the main body 852 to transition between the open position and the closed position.

In some examples, and as illustrated in FIGS. 7-8, the loading assist nut 850 comprises a locking mechanism 858 that is configured to selectively retain the receiver arm 856 in the closed position. In particular, in such examples, it can be necessary to selectively unlock, disable, release, and/or otherwise overcome the locking mechanism 858 prior to transitioning the receiver arm 856 from the closed position to the open position. When present, the locking mechanism 858 can comprise and/or be any suitable mechanism, examples of which include a latch, a clasp, a snap-fit mechanism, and/or a magnetic fastening mechanism.

While FIGS. 7-8 illustrate an example in which the receiver arm 856 remains attached to a remainder of the loading assist nut 850 when the receiver arm 856 is in the open position, this is not required of all examples. For example, it also is within the scope of the present disclosure that the receiver arm can be a separate component that can be completely removed from the main body to transition the receiver arm from the closed position to the open position.

FIGS. 4-6 and 9 additionally illustrate aspects of the torque transfer mechanism 830 of the capsule loading assist apparatus 800. In particular, in the example of FIGS. 4-6 and 9, the torque transfer mechanism 830 comprises a handle gear 832 configured to rotate in unison with the capsule translation knob 626 and a lead screw gear 834 operatively coupled to the lead screw 810 such that rotation of the handle gear 832 causes the lead screw 810 to rotate. In some examples, the lead screw gear 834 can be fixedly coupled to the lead screw 810.

The handle gear 832 can be connected to the capsule translation knob 626 in any suitable manner. In some examples, such as in the example of FIGS. 4-6 and 9, the handle gear 832 can be configured to be selectively coupled to the capsule translation knob 626, such as by sliding the handle gear 832 onto the capsule translation knob 626, by assembling the handle gear 832 around the capsule translation knob 626, etc. In other examples, the capsule translation knob can comprise and/or be the handle gear. For example, the capsule translation knob itself can comprise an outer surface that is ridged, textured, and/or otherwise shaped to rotationally engage another component of the torque transfer mechanism, such as the lead screw gear.

In some examples, such as in the example of FIGS. 4-6 and 9, the handle gear 832 directly engages the lead screw gear 834. Accordingly, in such an example, the first knob direction 627 is opposite to the first lead screw direction 811. This is not required of all examples, however, and it additionally is within the scope of the present disclosure that the torque transfer mechanism further can comprise one or more intermediate gears that operatively couple the handle gear and the lead screw gear to one another. In some such examples, the intermediate gear(s) can be configured such that the first knob direction is the same as the first lead screw direction or opposite to the first lead screw direction.

The features described herein with regard to any example can be combined with other features described in any one or more of the other examples, unless otherwise stated. For example, any one or more of the features of one capsule loading assist apparatus can be combined with any one or more features of another capsule loading assist apparatus. As another example, any one or more features of one delivery apparatus can be combined with any one or more features of another delivery apparatus.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims.

## Claims

1. A capsule loading assist apparatus (300; 800), comprising:
a lead screw (310; 810);
a loading assist nut (350; 850) engaged with the lead screw (310; 810) and comprising a capsule engagement surface (370; 870) configured to engage a proximal surface (434) of a delivery capsule (430) of a delivery apparatus (200; 400) to exert a distally directed loading assist force on the delivery capsule (430); and
a torque transfer mechanism (330; 830) configured to convey a torque from a handle (208; 408) of the delivery apparatus (200) to the lead screw (310; 810) to rotate the lead screw (310; 810);
wherein the capsule loading assist apparatus (300; 800) is configured such that rotating the lead screw (310; 810) in a first lead screw direction causes the loading assist nut (350; 850) to move along the lead screw (310; 810) in a distal direction.

2. The capsule loading assist apparatus (300; 800) of claim 1, wherein the loading assist nut (350; 850) comprises:
a main body (352; 852) that defines a lead screw receiver (354) that receives the lead screw (310; 810); and
a capsule shaft receiver (362; 862) configured to receive a capsule shaft (412) of the delivery apparatus (200; 400) that extends between the handle (208; 408) and the delivery capsule (430); and
wherein at least a portion of the main body (352; 852) extends between the lead screw receiver (354) and the capsule shaft receiver (362; 862).

3. The capsule loading assist apparatus (300; 800) of claim 2, wherein the main body (352; 852) defines at least a portion of the capsule engagement surface (370; 870); and/or wherein the main body (352; 852) defines at least a portion of the capsule shaft receiver (362; 862).

4. The capsule loading assist apparatus (300; 800) of any one of claims 2 to 3, wherein the loading assist nut (350; 850) further comprises a receiver arm (856) coupled to the main body (352; 852), wherein the receiver arm (856) defines at least a portion of the capsule shaft receiver (362; 862), and wherein the receiver arm (856) is configured to be selectively transitioned between an open position, in which at least a portion of the receiver arm (856) is spaced apart from the main (352; 852) body to allow the capsule shaft (412) to be inserted into or removed from the loading assist nut (350; 850), and a closed position, in which the loading assist nut (350; 850) is operative to exert the loading assist force on the delivery capsule (430) when the capsule shaft (412) is received within the capsule shaft receiver (362; 862).

5. The capsule loading assist apparatus (300; 800) of any one of claims 1 to 4, wherein the loading assist nut (350; 850) is configured to translate relative to the handle (208; 408) without rotating relative to the handle (208; 408) as the lead screw (310; 810) rotates.

6. The capsule loading assist apparatus (300; 800) of any one of claims 1 to 5, wherein the handle (208; 408) comprises:
a handle body (428); and
a capsule translation knob (426) configured to be selectively rotated relative to the handle body (428) in a first knob direction to translate the delivery capsule (430) in the distal direction relative to the handle body (428); and
wherein the torque transfer mechanism (330; 830) is configured to be selectively coupled to the capsule translation knob (426) such that rotation of the capsule translation knob (426) causes the lead screw (310; 810) to rotate; in particular wherein the torque transfer mechanism (330; 830) comprises:
a handle gear (832) configured to rotate in unison with the capsule translation knob (426); and
a lead screw gear (834) operatively coupled to the lead screw (310; 810); and
wherein rotation of the handle gear (832) causes the lead screw (310; 810) to rotate.

7. The capsule loading assist apparatus (300; 800) of any one of claims 1 to 6, further comprising a support shaft (320) that extends parallel to the lead screw (310; 810), wherein the support shaft (320) engages the loading assist nut (350; 850) to restrict the loading assist nut (350; 850) from rotating relative to the lead screw (310; 810); in particular wherein the loading assist nut (350; 850) comprises a support shaft receiver (382) that receives the support shaft (320).

8. The capsule loading assist apparatus (300; 800) of any one of claims 1 to 7, further comprising a proximal bearing (340) that supports a proximal end portion of the lead screw (310; 810).

9. The capsule loading assist apparatus (300; 800) of claim 8, further comprising a support shaft (322) that extends parallel to the lead screw (310; 810), wherein the support shaft (322) engages the loading assist nut (350; 850) to restrict the loading assist nut (350; 850) from rotating relative to the lead screw (310; 810), and wherein the proximal bearing (340) comprises a proximal bearing support shaft receiver (342) that receives the support shaft (322).

10. The capsule loading assist apparatus (300; 800) of any one of claims 8 to 9, wherein the proximal bearing (340) further is configured to support the handle (208; 408) relative to the lead screw (310; 810).

11. The capsule loading assist apparatus (300; 800) of any one of claims 1 to 8 and 10 when dependent on 8 further comprising a distal bearing (380) that supports a distal end portion of the lead screw (310; 810); in particular wherein the capsule loading assist apparatus (300; 800) further comprises a support shaft (322) that extends parallel to the lead screw (310; 810), wherein the support shaft (322) engages the loading assist nut (350; 850) to restrict the loading assist nut (350; 850) from rotating relative to the lead screw (310; 810), and wherein the distal bearing (380) comprises a distal bearing support shaft receiver (382) that receives the support shaft (322).

12. A capsule loading assist assembly, comprising:
a delivery apparatus (200) comprising a handle with a capsule translation knob (426), a delivery capsule (430), and a capsule shaft (412) extending between the handle (208; 408) and the delivery capsule (430);
a prosthetic heart valve (100; 500) supported by the delivery apparatus (200; 400); and
a capsule loading assist apparatus (300; 800) according to any one of claims 1 to 11.

13. A method comprising:
operatively coupling a delivery apparatus (200; 400) to a capsule loading assist apparatus (300; 800), wherein the delivery apparatus (200; 400) comprises a handle (208; 408) comprising a capsule translation knob (426), a delivery capsule (430), and a capsule shaft (412) extending between the handle (208; 408) and the delivery capsule (430), and wherein the capsule loading assist apparatus (300; 800) comprises a lead screw (310; 810), a loading assist nut (350; 850) engaged with the lead screw (310; 810) and a torque transfer mechanism (330; 830) configured to convey a torque from the handle (208; 408) of the delivery apparatus (200; 400) to the lead screw to rotate the lead screw (310; 810); and
rotating the capsule translation knob (426) in a first knob direction to translate the loading assist nut (350; 850) in a distal direction.

14. The method of claim 13, wherein the operatively coupling the delivery apparatus (200; 400) to the capsule loading assist apparatus (300; 800) comprises inserting the capsule shaft (412) into a capsule shaft receiver (362; 862) of the loading assist nut (350; 850).

15. The method of claim 14, wherein the loading assist nut (350; 850) comprises a main body (352; 852) and a receiver arm (856) coupled to the main body (352; 852), wherein the receiver arm (856) is configured to be selectively transitioned between an open position and a closed position, and wherein the inserting the capsule shaft (412) into the capsule shaft receiver (362; 862) comprises:
with the receiver arm (856) in the open position, positioning the capsule shaft (412) between the main body (352; 852) and the receiver arm (56); and
transitioning the receiver arm (856) from the open position to the closed position.

## Patentansprüche

1. Hilfsvorrichtung (300; 800) zum Laden einer Kapsel, umfassend:
eine Gewindespindel (310; 810);
eine Ladeunterstützungsmutter (350; 850), die mit der Gewindespindel (310; 810) in Eingriff steht und eine Kapseleingriffsfläche (370; 870) umfasst, die dazu ausgelegt ist, mit einer proximalen Oberfläche (434) einer Zuführkapsel (430) einer Zuführvorrichtung (200; 400) in Eingriff zu stehen, um eine distal gerichtete Ladeunterstützungskraft auf die Zuführkapsel (430) auszuüben; und einen Mechanismus zur Übertragung eines Drehmoments (330; 830), der dazu ausgelegt ist, ein Drehmoment von einem Griff (208; 408) der Zuführvorrichtung (200) auf die Gewindespindel (310; 810) zu übertragen, um die Gewindespindel (310; 810) zu drehen;
wobei die Hilfsvorrichtung (300; 800) zum Laden einer Kapsel dazu ausgelegt ist, dass ein Drehen der Gewindespindel (310; 810) in eine erste Gewindespindelrichtung bewirkt, dass sich die Ladeunterstützungsmutter (350; 850) in eine distale Richtung entlang der Gewindespindel (310; 810) bewegt.

2. Hilfsvorrichtung (300; 800) zum Laden einer Kapsel gemäß Anspruch 1, wobei die Ladeunterstützungsmutter (350; 850) umfasst:
einen Hauptkörper (351; 852), der einen Gewindespindelempfänger (354) definiert, der die Gewindespindel (310; 810) aufnimmt; und
einen Kapselschaftempfänger (362; 862), der dazu ausgelegt ist, einen Kapselschaft (412) der Zuführvorrichtung (200; 400), der sich zwischen dem Griff (208; 408) und der Zuführkapsel (430) erstreckt, aufzunehmen; und
wobei sich zumindest ein Abschnitt des Hauptkörpers (352; 852) zwischen dem Gewindespindelempfänger (354) und dem Kapselschaftempfänger (362; 862) erstreckt.

3. Hilfsvorrichtung (300; 800) zum Laden einer Kapsel gemäß Anspruch 2, wobei der Hauptkörper (352; 852) zumindest einen Abschnitt der Kapseleingriffsfläche (370; 870) definiert und/oder wobei der Hauptkörper (352; 852) zumindest einen Abschnitt des Kapselschaftempfängers (362; 862) definiert.

4. Hilfsvorrichtung (300; 800) zum Laden einer Kapsel gemäß einem der Ansprüche 2 bis 3, wobei die Ladeunterstützungsmutter (350; 850) ferner einen Empfängerarm (856) umfasst, der mit dem Hauptkörper (352; 852) verbunden ist, wobei der Empfängerarm (856) zumindest einen Abschnitt des Kapselschaftempfängers (362; 862) definiert, und wobei der Empfängerarm (856) dazu ausgelegt ist, selektiv zwischen einer offenen Position, in welcher zumindest ein Abschnitt des Empfängerarms (856) vom Hauptkörper (352; 852) beabstandet ist, um den Kapselschaft (412) zu erlauben, in die Ladeunterstützungsmutter (350; 850) eingeführt oder aus dieser entfernt zu werden, und einer geschlossenen Position, in welcher die Ladeunterstützungsmutter (350; 850) betriebsfähig ist, um die Ladeunterstützungskraft auf die Zuführkapsel (430) auszuüben, wenn der Kapselschaft (412) im Kapselschaftempfänger (362; 862) aufgenommen ist, überführt zu werden.

5. Hilfsvorrichtung (300; 800) zum Laden einer Kapsel gemäß einem der Ansprüche 1 bis 4, wobei die Ladeunterstützungsmutter (350; 850) dazu ausgelegt ist, sich relativ zum Griff (208; 408) zu verschieben, ohne sich relativ zum Griff (208; 408) zu drehen, wenn sich die Gewindespindel (310; 810) dreht.

6. Hilfsvorrichtung (300; 800) zum Laden einer Kapsel gemäß einem der Ansprüche 1 bis 5, wobei der Griff (208; 408) umfasst:
einen Griffkörper (428); und
einen Kapselverschiebeknopf (426) dazu ausgelegt, selektiv in eine erste Knopfrichtung relativ zu dem Griffkörper (428) gedreht zu werden, um die Zuführkapsel (430) in die distale Richtung relativ zu dem Griffkörper (428) zu verschieben; und
wobei der Mechanismus zur Übertragung eines Drehmoments (330; 830) dazu ausgelegt ist, selektiv mit dem Kapselverschiebeknopf (426) verbunden zu sein, so dass eine Rotation des Kapselverschiebeknopfes (426) bewirkt, dass sich die Gewindespindel (310; 810) dreht; wobei insbesondere der Mechanismus zur Übertragung eines Drehmoments (330; 830) umfasst:
ein Griffzahnrad (832) dazu ausgelegt, sich gemeinsam mit dem Kapselverschiebeknopf (426) zu drehen; und
ein Gewindespindelzahnrad (834), das funktionsfähig mit der Gewindespindel (310; 810) verbunden ist; und
wobei eine Drehung des Griffzahnrads (832) bewirkt, dass sich die Gewindespindel (310; 810) dreht.

7. Hilfsvorrichtung (300; 800) zum Laden einer Kapsel gemäß einem der Ansprüche 1 bis 6, ferner umfassend einen Stützschaft (320), der sich parallel zu der Gewindespindel (310; 810) erstreckt, wobei der Stützschaft (320) mit der Ladeunterstützungsmutter (350; 850) in Eingriff steht, um die Ladeunterstützungsmutter (350; 850) daran zu hindern, sich relativ zur Gewindespindel (310; 810) zu drehen; insbesondere wobei die Ladeunterstützungsmutter (350; 850) einen Stützschaftempfänger (382) umfasst, der den Stützschaft (320) aufnimmt.

8. Hilfsvorrichtung (300; 800) zum Laden einer Kapsel gemäß einem der Ansprüche 1 bis 7, ferner umfassend ein proximales Lager (340), das einen proximalen Endabschnitt der Gewindespindel (310; 810) stützt.

9. Hilfsvorrichtung (300; 800) zum Laden einer Kapsel gemäß Anspruch 8, ferner umfassend einen Stützschaft (322), der sich parallel zur Gewindespindel (310; 810) erstreckt, wobei der Stützschaft (322) in Eingriff mit der Ladeunterstützungsmutter (350; 850) steht, um die Ladeunterstützungsmutter (350; 850) in ihrer Drehung relativ zur Gewindespindel (310; 810) einzuschränken, und wobei das proximale Lager (340) einen proximalen Lagerstützschaftempfänger (342) umfasst, der den Stützschaft (322) aufnimmt.

10. Hilfsvorrichtung (300; 800) zum Laden einer Kapsel gemäß einem der Ansprüche 8 bis 9, wobei das proximale Lager (340) ferner dazu ausgelegt ist, den Griff (208; 408) relativ zur Gewindespindel (310; 810) zu stützen.

11. Hilfsvorrichtung (300; 800) zum Laden einer Kapsel gemäß einem der Ansprüche 1 bis 8 und 10, wenn abhängig von 8,
ferner umfassend ein distales Lager (380), das einen distalen Endabschnitt der Gewindespindel (310; 810) stützt; wobei insbesondere die Hilfsvorrichtung (300; 800) zum Laden einer Kapsel ferner einen Stützschaft (322) umfasst, der sich parallel zur Gewindespindel (310; 810) erstreckt, wobei der Stützschaft (322) in Eingriff mit der Ladeunterstützungsmutter (350; 850) steht, um die Ladeunterstützungsmutter (350; 850) in ihrer Drehung relativ zur Gewindespindel (310; 810) einzuschränken, und wobei das distale Lager (380) einen distalen Lagerstützschaftempfänger (342) umfasst, der den Stützschaft (322) aufnimmt.

12. Hilfsanordnung (300; 800) zum Laden einer Kapsel, umfassend:
eine Zuführvorrichtung (200) umfassend einen Griff mit einem Kapselverschiebeknopf (426), einer Zuführkapsel (430), und einem Kapselschaft (412), der sich zwischen dem Griff (208; 408) und der Zuführkapsel (430) erstreckt;
eine prothetische Herzklappe (100; 500), die durch die Zuführvorrichtung (200; 400) gehalten wird; und
eine Hilfsvorrichtung (300; 800) zum Laden einer Kapsel gemäß einem der Ansprüche 1 bis 11.

13. Verfahren, umfassend:
Operatives Verbinden einer Zuführvorrichtung (200; 400) mit einer Hilfsvorrichtung (300; 800) zum Laden einer Kapsel, wobei die Zuführvorrichtung (200; 400) einen Griff (208; 408) umfasst, der einen Kapselverschiebeknopf (426), eine Zuführkapsel (430), und einen Kapselschaft (412), der sich zwischen dem Griff (208; 408) und der Zuführkapsel (430) erstreckt, umfasst, und wobei die Hilfsvorrichtung (300; 800) zum Laden einer Kapsel eine Gewindespindel (310; 810), eine Ladeunterstützungsmutter (350; 850), die in Eingriff mit der Gewindespindel (310; 810) steht, und einen Mechanismus zur Übertragung eines Drehmoments (330; 830), der dazu ausgelegt ist, ein Drehmoment vom Griff (208; 408) der Zuführvorrichtung (200; 400) auf die Gewindespindel (310; 810) zu übertragen, um die Gewindespindel (310; 810) zu drehen, umfasst; und
Drehen des Kapselverschiebeknopfes (426) in eine erste Knopfrichtung, um die Ladeunterstützungsmutter (350; 850) in eine distale Richtung zu verschieben.

14. Verfahren gemäß Anspruch 13, wobei das operative Verbinden der Zuführvorrichtung (200; 400) mit der Ladeunterstützungsmutter (350; 850) das Einführen des Kapselschafts (412) in einen Kapselschaftempfänger (362; 862) der Ladeunterstützungsmutter (350; 850) umfasst.

15. Verfahren gemäß Anspruch 14, wobei die Ladeunterstützungsmutter (350; 850) einen Hauptkörper (352; 852) und einen Empfängerarm (856), der mit dem Hauptkörper (352; 852) verbunden ist, umfasst, wobei der Empfängerarm (856) dazu ausgelegt ist, selektiv zwischen einer offenen Position und einer geschlossenen Position überführt zu werden, und wobei das Einführen des Kapselschafts (412) in den Kapselschaftempfänger (362; 862) umfasst:
Positionieren des Kapselschafts (412) zwischen dem Hauptkörper (352; 852) und dem Empfängerarm (56), wenn sich der Empfängerarm (856) in der offenen Position befindet; und
Überführen des Empfängerarms (856) von der offenen in die geschlossene Position.

## Revendications

1. Appareil d'assistance (300 ; 800) au chargement de capsule, comprenant :
une vis-mère (310 ; 810) ;
un écrou d'assistance (350 ; 850) au chargement en prise avec la vis-mère (310 ; 810) et comprenant une surface de mise en prise (370 ; 870) avec la capsule conçue pour venir en prise avec une surface proximale (434) d'une capsule de pose (430) d'un appareil de pose (200 ; 400) afin d'exercer une force d'assistance au chargement dirigée de manière distale sur la capsule de pose (430) ; et
un mécanisme de transfert de couple (330 ; 830) conçu pour transmettre un couple à partir d'une poignée (208 ; 408) de l'appareil de pose (200) à la vis-mère (310 ; 810) afin de faire tourner la vis-mère (310 ; 810) ;
l'appareil d'assistance (300 ; 800) au chargement de capsule étant conçu de telle sorte que la rotation de la vis-mère (310 ; 810) dans un premier sens de vis-mère amène l'écrou d'assistance (350 ; 850) au chargement à se déplacer le long de la vis-mère (310 ; 810) dans un sens distal.

2. Appareil d'assistance (300 ; 800) au chargement de capsule selon la revendication 1, l'écrou d'assistance (350 ; 850) au chargement comprenant :
un corps principal (352 ; 852) qui définit un récepteur (354) de vis-mère qui reçoit la vis-mère (310 ; 810) ; et
un récepteur (362 ; 862) de tige de capsule conçu pour recevoir une tige (412) de capsule de l'appareil de pose (200 ; 400) qui s'étend entre la poignée (208 ; 408) et la capsule de pose (430) ; et
au moins une partie du corps principal (352 ; 852) s'étendant entre le récepteur (354) de vis-mère et le récepteur (362 ; 862) de tige de capsule.

3. Appareil d'assistance (300 ; 800) au chargement de capsule selon la revendication 2, le corps principal (352 ; 852) définissant au moins une partie de la surface de mise en prise (370 ; 870) avec la capsule ; et/ou le corps principal (352 ; 852) définissant au moins une partie du récepteur (362 ; 862) de tige de capsule.

4. Appareil d'assistance (300 ; 800) au chargement de capsule selon l'une quelconque des revendications 2 à 3, l'écrou d'assistance (350 ; 850) au chargement comprenant en outre un bras récepteur (856) accouplé au corps principal (352 ; 852), le bras récepteur (856) définissant au moins une partie du récepteur (362 ; 862) de tige de capsule et le bras récepteur (856) étant conçu pour passer sélectivement entre une position ouverte, dans laquelle au moins une partie du bras récepteur (856) est écartée du corps principal (352 ; 852) afin de permettre l'insertion de la tige (412) de capsule dans l'écrou d'assistance (350 ; 850) au chargement ou son retrait à partir de celui-ci, et une position fermée, dans laquelle l'écrou d'assistance (350 ; 850) au chargement est opérationnel pour exercer la force d'assistance au chargement sur la capsule de pose (430) lorsque la tige (412) de capsule est logée à l'intérieur du récepteur (362 ; 862) de tige de capsule.

5. Appareil d'assistance (300 ; 800) au chargement de capsule selon l'une quelconque des revendications 1 à 4,
l'écrou d'assistance (350 ; 850) au chargement étant conçu pour effectuer une translation par rapport à la poignée (208 ; 408) sans tourner par rapport à la poignée (208 ; 408) lorsque la vis-mère (310 ; 810) tourne.

6. Appareil d'assistance (300 ; 800) au chargement de capsule selon l'une quelconque des revendications 1 à 5,
la poignée (208 ; 408) comprenant :
un corps (428) de poignée ; et
un bouton de translation (426) de capsule conçu pour être sélectivement tourné par rapport au corps (428) de poignée dans un premier sens de bouton afin que la capsule de pose (430) effectue une translation dans le sens distal par rapport au corps (428) de poignée ; et
le mécanisme de transfert de couple (330 ; 830) étant conçu pour être sélectivement accouplé au bouton de translation (426) de capsule de telle sorte que la rotation du bouton de translation (426) de capsule amène la vis-mère (310 ; 810) à tourner ; en particulier le mécanisme de transfert de couple (330 ; 830) comprenant :
un engrenage (832) de poignée conçu pour tourner à l'unisson avec le bouton de translation (426) de capsule ; et
un engrenage (834) de vis-mère accouplé de manière fonctionnelle à la vis-mère (310 ; 810) ; et
la rotation de l'engrenage (832) de poignée amenant la vis-mère (310 ; 810) à tourner.

7. Appareil d'assistance (300 ; 800) au chargement de capsule selon l'une quelconque des revendications 1 à 6,
comprenant en outre une tige support (320) qui s'étend parallèlement à la vis-mère (310 ; 810), la tige support (320) venant en prise avec l'écrou d'assistance (350 ; 850) au chargement afin d'empêcher la rotation de l'écrou d'assistance (350 ; 850) au chargement par rapport à la vis-mère (310 ; 810) ; en particulier l'écrou d'assistance (350 ; 850) au chargement comprenant un récepteur (382) de tige support qui reçoit la tige support (320).

8. Appareil d'assistance (300 ; 800) au chargement de capsule selon l'une quelconque des revendications 1 à 7,
comprenant en outre un palier proximal (340) qui supporte une partie d'extrémité proximale de la vis-mère (310 ; 810).

9. Appareil d'assistance (300 ; 800) au chargement de capsule selon la revendication 8, comprenant en outre
une tige support (322) qui s'étend parallèlement à la vis-mère (310 ; 810), la tige support (322) venant en prise avec l'écrou d'assistance (350 ; 850) au chargement afin d'empêcher la rotation de l'écrou d'assistance (350 ; 850) au chargement par rapport à la vis-mère (310 ; 810) et le palier proximal (340) comprenant un récepteur (342) de tige support de palier proximal qui reçoit la tige support (322).

10. Appareil d'assistance (300 ; 800) au chargement de capsule selon l'une quelconque des revendications 8 à 9,
le palier proximal (340) étant en outre conçu pour supporter la poignée (208 ; 408) par rapport à la vis-mère (310 ; 810).

11. Appareil d'assistance (300 ; 800) au chargement de capsule selon l'une quelconque des revendications 1 à 8 et 10 lorsqu'elle dépend de 8, comprenant en outre un palier distal (380) qui supporte une partie d'extrémité distale de la vis-mère (310 ; 810) ; en particulier l'appareil d'assistance (300 ; 800) au chargement de capsule comprenant en outre une tige support (322) qui s'étend parallèlement à la vis-mère (310 ; 810), la tige support (322) venant en prise avec l'écrou d'assistance (350 ; 850) au chargement afin d'empêcher la rotation de l'écrou d'assistance (350 ; 850) au chargement par rapport à la vis-mère (310 ; 810) et le palier distal (380) comprenant un récepteur (382) de tige support de palier distal qui reçoit la tige support (322).

12. Ensemble d'assistance au chargement de capsule, comprenant :
un appareil de pose (200) comprenant une poignée pourvue d'un bouton de translation (426) de capsule, une capsule de pose (430) et une tige (412) de capsule s'étendant entre la poignée (208 ; 408) et la capsule de pose (430) ;
une valvule cardiaque prothétique (100 ; 500) supportée par l'appareil de pose (200 ; 400) ;
et
un appareil d'assistance (300 ; 800) au chargement de capsule selon l'une quelconque des revendications 1 à 11.

13. Procédé comprenant :
l'accouplement fonctionnel d'un appareil de pose (200 ; 400) à un appareil d'assistance (300 ; 800) au chargement de capsule, l'appareil de pose (200 ; 400) comprenant une poignée (208 ; 408) comprenant un bouton de translation (426) de capsule, une capsule de pose (430) et une tige (412) de capsule s'étendant entre la poignée (208 ; 408) et la capsule de pose (430) et l'appareil d'assistance (300 ; 800) au chargement de capsule comprenant une vis-mère (310 ; 810), un écrou d'assistance (350 ; 850) au chargement en prise avec la vis-mère (310 ; 810) et un mécanisme de transfert de couple (330 ; 830) conçu pour transmettre un couple à partir de la poignée (208 ; 408) de l'appareil de pose (200 ; 400) à la vis-mère afin de faire tourner la vis-mère (310 ; 810) ; et
la rotation du bouton de translation (426) de capsule dans un premier sens de bouton afin que l'écrou d'assistance (350 ; 850) au chargement effectue une translation dans un sens distal.

14. Procédé selon la revendication 13, l'accouplement fonctionnel de l'appareil de pose (200 ; 400) à l'appareil d'assistance (300 ; 800) au chargement de capsule comprenant l'insertion de la tige (412) de capsule dans un récepteur (362 ; 862) de tige de capsule de l'écrou d'assistance (350 ; 850) au chargement.

15. Procédé selon la revendication 14, l'écrou d'assistance (350 ; 850) au chargement comprenant un corps principal (352 ; 852) et un bras récepteur (856) accouplé au corps principal (352 ; 852), le bras récepteur (856) étant conçu pour passer sélectivement entre une position ouverte et une position fermée et l'insertion de la tige (412) de capsule dans le récepteur (362 ; 862) de tige de capsule comprenant :
le bras récepteur (856) étant en position ouverte, le positionnement de la tige (412) de capsule entre le corps principal (352 ; 852) et le bras récepteur (56) ; et
le passage du bras récepteur (856) à partir de la position ouverte vers la position fermée.
